# EUROPEAN PATENT APPLICATION

(11) **EP 2 015 028 A2**
(43) Date of publication of application: **14.01.2009**
(21) Application number: 08154153.4
(22) Date of filing: 07.04.2008
(51) Int. Cl.: G01C 22/00

(54) **Measuring method, measuring apparatus and computer readable information recording medium for determining a walking and moving state of a person**

(30) Priority: 13.07.2007 JP 2007184579
(71) Applicant: FUJITSU LIMITED, Kawasaki-shi, Kanagawa 211-8588 (JP)
(72) Inventor: Kasama, Koichiro c/o FUJITSU LIMITED, Kawasaki-shi, Kanagawa 211-8588 (JP); Kanno, Hiroshi c/o FUJITSU LIMITED, Kawasaki-shi, Kanagawa 211-8588 (JP); Yamamoto, Nobuhisa c/o FUJITSU LIMITED, Kawasaki-shi, Kanagawa 211-8588 (JP)
(74) Representative: Desormiere, Pierre-Louis

(57) **Abstract**

A measuring apparatus (100) has a walking determining part (110) configured to determine a walking state of a to-be-measured person, a moving state determining part (120) configured to determine a moving state in a specific direction of the to-be-measured person, a measurement result output determining part (150) configured to determine whether or not measurement information concerning a movement of the to-be-measured person is to be output, based on a determination result of the walking state of the to-be-measured person by said walking determining part (110) and a determination result of the moving state of the to-be-measured person by the moving state determining part (120).

## Description

### BACKGROUND OF THE INVENTION

### 1. Field of the Invention

The present invention relates to a measuring method, a measuring apparatus and a computer readable information recording medium, and in particular, to a measuring method for measuring a moving state of a to-be-measured person, a measuring apparatus carrying out the method and a computer readable information recording medium storing a program for causing a computer to carry out the method.

### 2. Description of the Related Art

Recently, a measurement of an exercise amount during walking is required. For this purpose, a cellular phone may be provided with an apparatus for measuring an exercise amount.

For example, Japanese Laid-Open Patent Application 11-347020 proposes an expended calorie calculating apparatus for calculating calories expended as a result of a to-be-measured person making an exercise.

In this expended calorie calculating apparatus, a ground height detecting part, a walking detecting part, a walking determining part, an expended calorie calculating part, an expended calorie outputting part are provided. The ground height detecting part has an ultrasonic transmitting part and an ultrasonic receiving part. Ultrasonic waves transmitted by the ultrasonic transmitting part are reflected by a walking surface, and then, is returned to the ultrasonic receiving part. A time required therefor is measured and thus, a height from the ground is measured. A change in the height from the ground is detected, and thus, it is determined whether the to-be-measured person is currently going up or down a hill or stairs.

Japanese Laid-Open Patent Application 2006-220653 discloses another related art.

### SUMMARY OF THE INVENTION

An object of the present invention is to provide a configuration such that, even when the configuration is provided in a cellular phone or such, an exercise amount of a to-be-measured person can be appropriately obtained.

According to the present invention, it is determined whether or not a to-be-measured person is currently walking, and a moving rate of the to-be-measured person is measured while it is determined that the to-be-measured person is currently walking. Then, based on the moving rate of the to-be-measured person in a specific direction while it is determined that the to-be-measured person is currently walking, it is determined whether measurement information concerning the movement of the to-be-measured person obtained during the time is to be output.

In this configuration, first it is determined whether or not the to-be-measured person is currently walking. Also, when it is determined that the to-be-measured person is currently walking, a moving rate of the to-be-measured person in a specific direction is measured. Then, based on the moving rate of the to-be-measured person in the specific direction while it is determined that the to-be-measured person is currently walking, it is determined whether measurement information concerning the movement of the to-be-measured person obtained when the to-be-measured person is walking is to be output. Therefore, if the to-be-measured person moves with the use of an elevator for example, it is determined that the to-be-measured person is not currently walking. Therefore, in this case, even though the to-be-measured person moves in a specific direction (i.e., a vertical direction by means of the elevator), it is determined that the measurement result is not to be output.

On the other hand, when the to-be-measured person goes up/down stairs, it is determined that the to-be-measured person is currently walking, and thus, it is determined that the measurement result is to be output. Thus, when the to-be-measured person moves but does not go up/down stairs, the measurement result is not output. On the other hand, when the to-be-measured person goes up/down stairs, the measurement result is positively output.

Thus, according to the present invention, it is determined whether a measurement result is to be output, based on a determination result of whether a to-be-required person goes up/down stairs. Therefore, it is possible to obtain an exercise amount of the to-be-measured person concerning going up/down stairs. As a result, it is possible to obtain an exercise amount of the to-be-measured person more precisely.

Since the present invention determines whether a to-be-measured person is currently going up/down stairs by measuring a moving rate of the to-be-measured person in a specific direction, it is possible to appropriately obtain an exercise amount of the to-be-measured person even when a corresponding apparatus is provided in a cellular phone or such.

### BRIEF DESCRIPTION OF THE DRAWINGS

FIG. 1 shows a block diagram of a measuring apparatus in a first embodiment of the present invention;
FIG. 2 shows an operation flow chart of the measuring apparatus in the first embodiment of the present invention;
FIG. 3 shows a detailed operation flow chart of a moving rate measuring process in each embodiment of the present invention;
FIG. 4 shows a block diagram of a measuring apparatus in a second embodiment of the present invention;
FIG. 5 shows an operation flow chart of the measuring apparatus in the second embodiment of the present invention;
FIG. 6 shows a block diagram of a measuring apparatus in a third embodiment of the present invention;
FIG. 7 shows an operation flow chart of the measuring apparatus in the third embodiment of the present invention;
FIG. 8 illustrates specific configuration examples for obtaining respective measurement values in each embodiment;
FIG. 9 shows a hardware block diagram for when information processing in each embodiment is carried out by a computer; and
FIG. 10 shows a hardware configuration example of a cellular phone as one embodiment of the present invention.

### DETAILED DESCRIPTION OF THE PREFERRED EMBODIMENTS

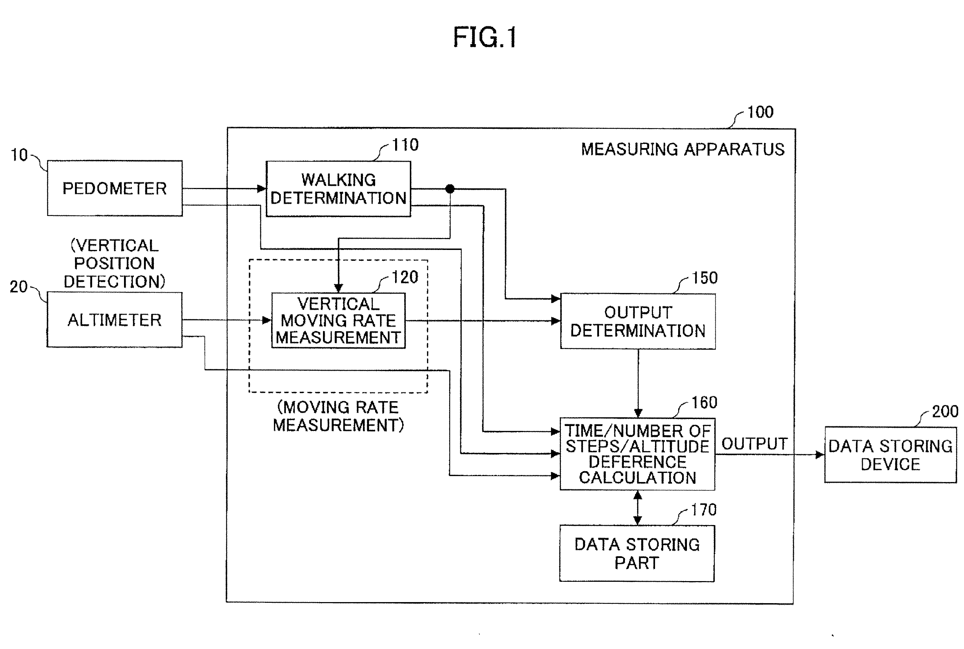

FIG. 1 shows a block diagram of a measuring apparatus 100 in a first embodiment of the present invention.

The measuring apparatus 100 in the embodiment is used in such a manner that it is provided in the inside of a cellular phone. In this case, in the inside of the cellular phone, as shown in FIG. 1, also a pedometer 10 configured to measure the number of steps of a to-be-measured person, an altimeter 20 configured to measure an altitude at which the measuring apparatus is located, and a data storing device 200, are provided.

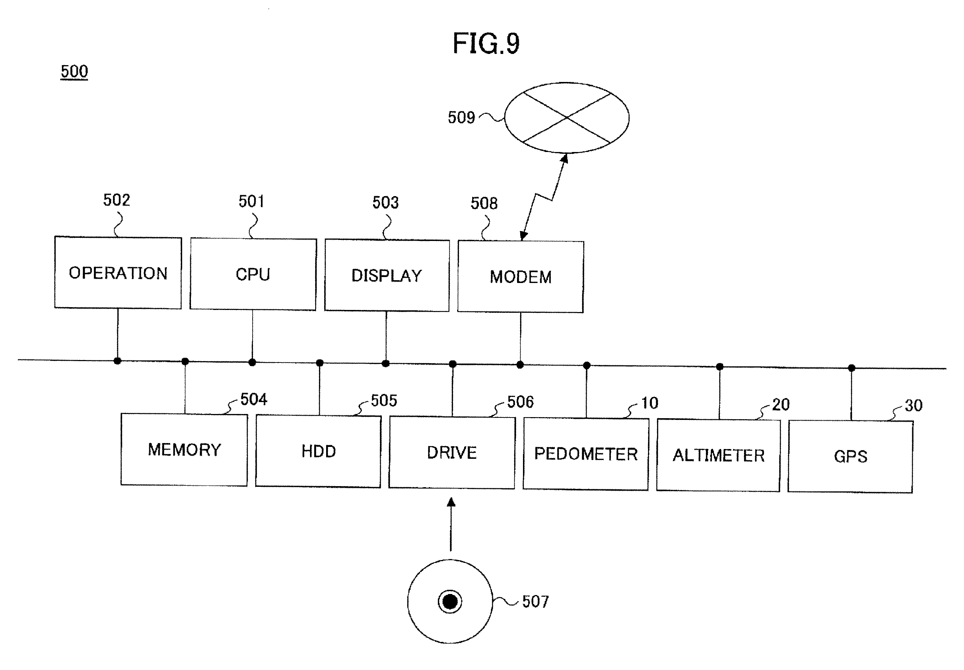

Further, as will be described later with reference to FIG. 9, operation carried out by the measuring apparatus 100 is carried out as a result of a computer executing instructions written in a program which is provided in such a manner that the program is stored in a computer readable information recording medium.

As shown in FIG. 1, the measuring apparatus 100 has a walking determining part 110 which processes an output signal from the pedometer 10 to determine whether a to-be-measured person is currently walking. Further, the measuring apparatus 100 has a vertical moving rate measuring part 120 which processes respective output signals from the walking determining part 110 and the altimeter 20, to detect a change in an altitude at which the to-be-measured person is thus walking, and measure a moving amount per a predetermined time (which will be referred to as a 'moving rate') in a vertical direction.

Further, the measuring apparatus 100 has an output determining part 150. The output determining part 150 processes respective output signals from the walking determining part 110 and the vertical moving rate measuring part 120 to determine whether the to-be-measured person is currently going up/down stairs. Then, the output determining part 150 provides an instruction to a time/number of steps/altitude difference calculating part 160 such that, calculation results of the time/number of steps/altitude difference calculating part 160, described later, are output to the data storing device 200, only when the output determining part 150 determines that the to-be-measured person is currently going up/down stairs.

The time/number of steps/altitude difference calculating part 160, which the measuring apparatus 100 also has, processes respective output signals from the pedometer 10, the altimeter 20, the walking determining part 110 and the output determining part 150, to calculate a time elapsed during when the to-be-measured person is going up/down stairs, the number of steps during this time and an altitude difference during this time. Then, the time/number of steps/altitude difference calculating part 160 outputs these calculation results to the data storing device 200.

Further, the measuring apparatus 100 includes a data storing part 170 which is configured to store data used by the time/number of steps/altitude difference calculating part 160 for the calculation.

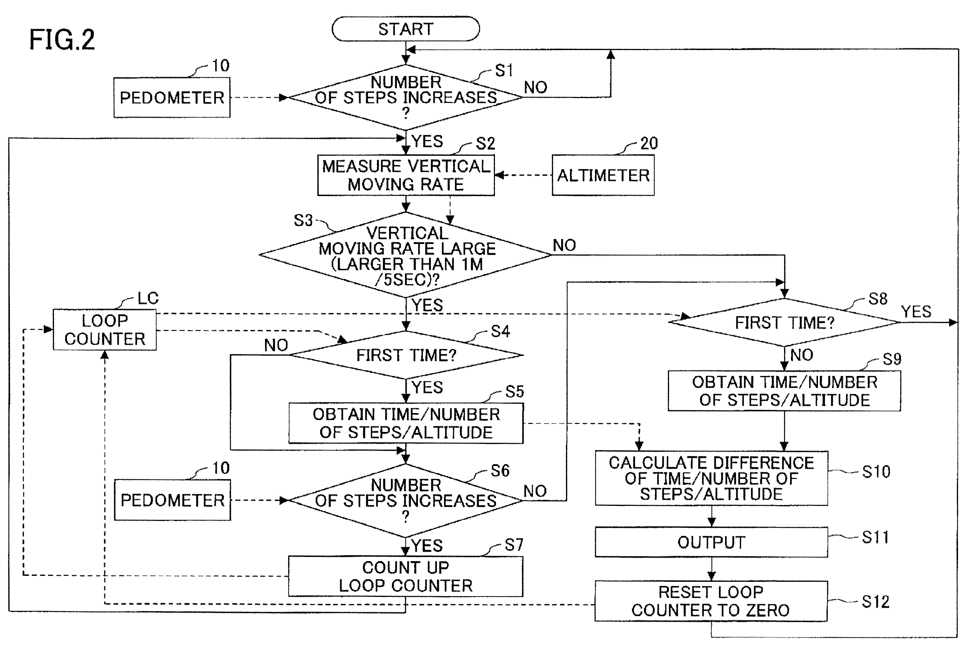

FIG. 2 shows a procedure carried out by the measuring apparatus 100.

It is noted that, here, it is assumed that a to-be-measured person carries the cellular phone having the measuring apparatus 100, the pedometer 10 and the altimeter 20, provided in the inside thereof.

In FIG. 2, the walking determining part 110 reads an output signal of the pedometer 10 in step S1, and compares the thus-obtained output count value of the number of steps from the pedometer 10 with an output count value of the number of steps previously obtained from the pedometer 10. Then, when the comparison result is that the output count value of the number of steps has increased (YES), step S2 is then carried out. On the other hand, when the comparison result is that the output count value of the number of steps has not increased (NO in step S1), the comparison process of step S1 is repeated at a predetermined interval.

It is noted that, the pedometer 10 is provided in the inside of the cellular phone, and the to-be-measured person carries the cellular phone. Accordingly, the output signal of the pedometer 10 indicates the number of steps of the to-be-measured person himself or herself. During when the to-be-measured person is currently walking, the output count value of the number of steps of the pedometer 10 gradually increases accordingly. Thus, the walking determining part 110 detects the increase in the output count value of the number of steps by receiving the output signal of the pedometer 10 which indicates the output count value of the number of steps of the pedometer 10. Thus, the walking determining part 110 can detect a state of the to-be-measured person currently walking.

In step S2, the vertical moving rate measuring part 120 receives an output signal of the altimeter 20, and obtains a difference value of altitude during a predetermined time, for example, 5 seconds. Thus, the vertical moving rate measuring part 120 obtains a moving rate in a vertical direction.

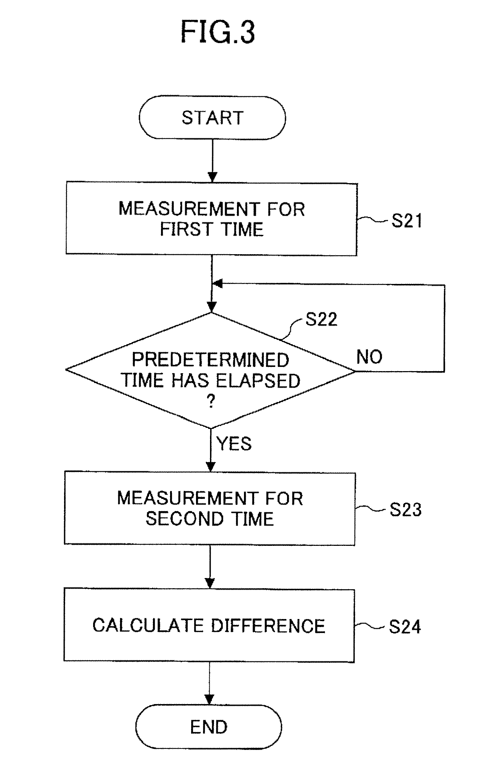

It is noted that, the output signal of the altimeter 20 indicates an altitude measurement value. When the to-be-required person goes up stairs for example, or goes down stairs, an altitude of the to-be-measured person gradually increases or decreases accordingly. As a result, the altitude measurement value gradually increases or decreases accordingly. The vertical moving rate measuring part 120 receives the output signal indicating the altitude measurement value from the altimeter 30, and thus, can detect that the altitude of the to-be-measured person thus gradually increases or decreases. It is noted that, the operation in step S2 will be described in more detail later with reference to FIG. 3.

Then, in step S3, an absolute value of the moving rate in the vertical direction thus obtained in step S2, i.e., an absolute value of the difference in altitude measured during 5 seconds is compared with a threshold, for example, 1 meter. In this case, when the altitude measured has increased equal to or larger than 1 m during 5 seconds, it is determined that the to-be-measured person goes up stairs. When the altitude measured decreases larger than 1 m during 5 seconds, it is determined that the to-be-measured person goes down stairs.

It is noted that, before reaching step S3, it is determined in step S1 whether the to-be-measured person is currently walking. Assuming that, for example, the to-be-measured person is currently taking an elevator, an escalator or such, and is not currently walking, the altitude of the to-be-measured person changes. However, in such a case, it is detected in step S1 that the to-be-measured person is thus not currently walking. Therefore, in this case, step S3 is not reached. As a result, an erroneous determination that the to-be-measured person is currently going up/down stairs can be positively avoided.

Further, a principle of the pedometer 10 to detect whether a to-be-measured person is currently walking is the same as well-known principle of a common pedometer, and thus, a further description thereof is omitted.

When the comparison result in step S3 is YES, i.e., when the absolute value of the moving rate in the vertical direction is equal to or larger than "1m/5s" (i.e., 1 meter per 5 seconds), it is finally determined that the to-be-measured person is currently going up/down stairs.

That is, when it is determined in step S1 that the to-be-measured person is currently walking, and also, it is determined in step S3 that the to-be-measured person is currently going up/down, it is determined that the to-be-measured person is currently going up/down stairs.

When the comparison result in step S3 is NO, i.e., when the absolute value of the moving rate in the vertical direction is smaller than "1m/5s" (i.e., 1 meter per 5 seconds), it is determined that the moving rate in the vertical direction is not so large as to determine that the to-be-measured person is currently going up/down stairs. As a result, step S8 is carried out then. Operation in step S8 will be described later.

When the comparison result in step S3 YES, step S4 is carried out then. In step S4, first, it is determined whether execution of step S4 is for the first time. In order to determine how many times S4 step has been executed, a loop counter LC is provided in the measuring apparatus 100. The loop counter LC is incremented by one in step S7 as will be described later. The loop counter LC is reset to zero in step S12, as will be described later, each time when a finish of the to-be-measured person's going up/down stairs is detected.

It is noted that, in the flow of FIG. 2, after it is determined that the to-be-measured person starts going up/down stairs as a result of the comparison result of step S1 being YES and also the comparison result of step S3 being YES, loop operation of steps S2, S3, S4, S5, S6, S7 and then again S2 is repeated until the comparison result of step S1 or the comparison result of step S2 becomes NO. The loop counter LC is incremented by one in step S7 each time the loop operation is executed. Therefore, the count value of the loop counter LC indicates the number of times of repetition of the loop operation. Further, when it is determined that the to-be-measured person's going up/down has finished, the count value of the loop counter LC is reset to zero in step S12. Therefore, the count value of the loop counter LC indicates the number of times of repetition of the loop operation since when the to-be-measured person has started going up/down stairs.

In step S4, it is determined whether step S4 is currently carried out for the first time. When the determination result of step S4 is YES, i.e., when the count value of the loop counter LC is zero, this means that the current loop operation is of the first time, and thus, this means that the to-be-measured person currently has started going up/down stairs.

In this case, step S5 is carried out in which a current time, the current output count value of the number of steps indicated by the output signal of the pedometer 10, and the current altitude measurement value indicated by the output signal of the altimeter 20, are stored in the data storing part 170 as values at a start of going up/down stairs. Information of the current time is obtained from a digital clock of the cellular phone provided with the measuring apparatus 100.

When the determination result of step S4 is NO, this means that the current execution of step S4 is for the second time or more, and thus, this means that the values at the start of going up/down stairs have already been stored in the storing part 170 in the loop operation of the first time. In this case, step S5 is skipped, and then, step S6 is carried out.

In step S6, the output count value of the number of steps indicated by the output signal of the pedometer 10 is read again. Then, the thus-read output count value of the number of steps is compared with the output value of the number of steps previously read. This output value of the number of steps previously read to be compared there is, for the loop operation of the first time, the output count value of the number of steps obtained in step S1. The output value of the number of steps previously read to be compared there is, for the loop operation of the second time or more, the output count value of the number of steps obtained in step S6 of the previous loop operation.

When the comparison result of step S6 is that the output value of the number of steps currently obtained is larger than the output value of the number of steps previously obtained, i.e., that the output value of the number of steps has increased, step S7 is carried out then. In step S7, the loop counter LC is incremented by one, and then, from step S2, the above-mentioned loop operation is repeated.

When the comparison result of step S6 is NO, i.e., the output value of the number of steps currently obtained is not lager than the output value of the number of steps previously obtained, this means that the number of steps has not increased, and thus, it is determined that the to-be-measured person has finished going up/down stairs, or when the comparison result of step S3 is NO in the loop operation of the second time or more, i.e., the absolute value of the moving rate in the vertical direction is smaller than 1m/5s, it is determined that the to-be-measured person has finished going up/down stairs.

In step S6, the output count value of the number of steps is compared between the current time and the previous time. During this time, a change amount (i.e., difference value) of altitude is measured for 5 seconds in step S3. This means that, at least 5 seconds have elapsed from the time at which the output count value of the number of steps is read previously. When the comparison result of step S6 is NO, this means that the output count value of the number of steps has not increased during the 5 seconds. That is, during 5 seconds, the to-be-measured person has not walked any step. In such a case, it is determined that the to-be-measured person has finished walking, i.e., has finished going up/down stairs in this case.

When the comparison result of step S3 in the loop operation of the second time or more is NO, it is determined that the current moving rate in the vertical direction is not so large as to determine that the to-be-measured person is currently going up/down stairs. As a result, it is determined that the to-be-measured person's going up/down stairs has been finished. In this case, since it has been determined in the previous step S6 that the output value of the number of steps has increased, it is determined that the to-be-measured person has still continued walking, with finishing going up/down stairs, and instead, with starting walking on a horizontal plane.

When thus the comparison result of step S3 or S6 is NO, i.e., it is determined that the to-be-measured person is not currently going up/down stairs or that the to-be-measured person has finished going up/down stairs, the count value of the loop counter LC is read in step S8. When the count value of the loop counter LC is zero in step S8, the current loop operation is of the first time. In this case, step S1 is then carried out. In step S1, it is determined whether the output count value of the number of steps has increased or not.

Next, when the determination result of step S8 is NO, respective values of time/number of steps/altitude are obtained again as values at a finish of going up/down stairs in step S9. That is, the same as in step S5 mentioned above, the current time, the current output count value of the number of steps indicated by the output signal of the pedometer 10 and the current altitude measurement value indicated by the output signal of the altimeter 20 are stored as the values at a finish of going up/down stairs in the data storing part 170.

Next, in step S10, differences between the respective values of time/number of steps/altitude at the start of going up/down stairs obtained in step S5 and the respective values of time/number of steps/altitude at the finish of going up/down stairs obtained in step S9 are calculated. The thus-obtained difference values are output to the data storing device 200 in step S11. After that, the loop counter LC is reset to zero (step S12), and the current process is finished. After that, step S1 is again carried out, and then, the above-mentioned process is repeated.

When the determination result of step S8 is YES, this means that the current loop operation is of the first time, and the process of step S1 is carried out again.

When step S8 is carried out directly after step S3 in the loop operation of the first time, this means that no going up/down stairs has been started. In this case, no values of time/number of steps/altitude have been obtained. Therefore, in this case, there is no meaning to carry out step S9 of obtaining respective values of time/number of steps/altitude at a finish of going up/down stairs. Therefore, in this case, the loop counter LC is read in step S8, which results in YES in this case since this is the loop operation of the first time, and step S1 is then carried out without carrying out step S9. Further, a situation that, in the loop operation of the first time, step S8 is carried out directly after step S6, is a situation that, this is immediately after respective values of time/number of steps/altitude have been obtained in step S5. Even if respective values of time/number of steps/altitude have been obtained in step S9 in such a situation, respective difference values of time/number of steps/altitude then calculated in step S10 become approximately zeros accordingly. Therefore, also in this case, the loop counter LC is read in step S8, which results in YES in this case since this is the loop operation of the first time, step S1 is then carried out without carrying out step S9.

Next, with reference to FIG. 3, a vertical moving rate measuring process (step S2) of the flow of FIG. 2 will be described in detail.

First, in step S21, altitude measurement for the first time is carried out.

After that, in step S22, time elapse is determined. That is, when a predetermined time, in this embodiment, 5 seconds, has elapsed, a next step S23 is carried out.

In step S23, altitude measurement for the second time is carried out.

In step S24, a difference between an altitude measurement value obtained in step S21 and an altitude measurement value obtained in step S23 is calculated as the moving rate in the vertical direction of the to-be-measured person.

Thus, the moving rate in the vertical direction of the to-be-measured person, i.e., a moving amount during 5 seconds, is obtained.

Next, a second embodiment according to the present invention will be described.

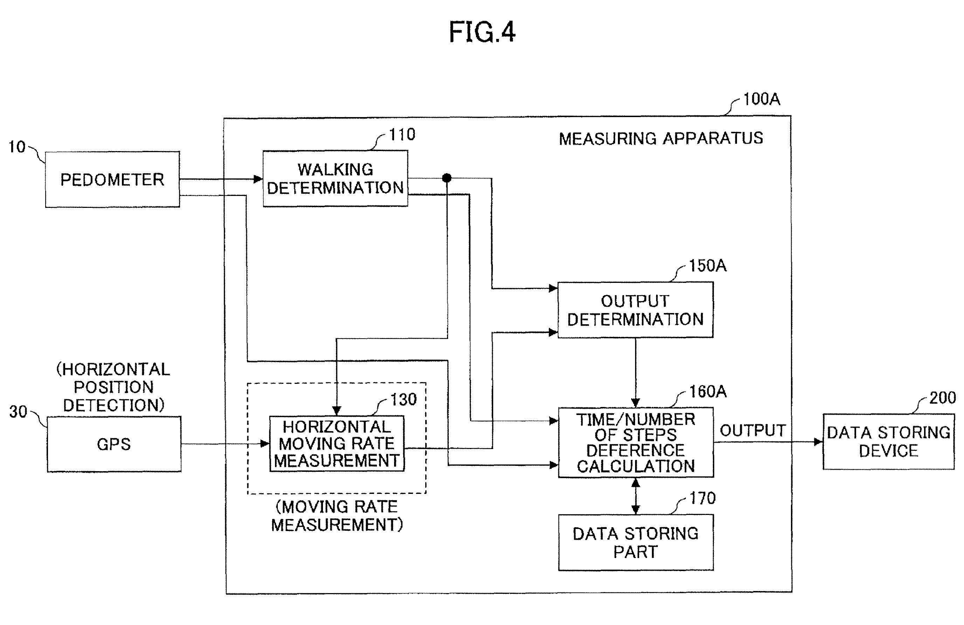

FIG. 4 shows a block diagram of a measuring apparatus 100A in the seconds embodiment of the present invention.

The same as the measuring apparatus 100 in the first embodiment, the measuring apparatus 100A in the embodiment is used in such a manner that it is provided in the inside of a cellular phone. In this case, in the inside of the cellular phone, as shown in FIG. 4, also a pedometer 10 configured to measure the number of steps of a to-be-measured person, a GPS 30 configured to measure a horizontal position at which the measuring apparatus 100A is located, and a data storing device 200, are provided.

Further, as will be described later with reference to FIG. 9, operation carried out by the measuring apparatus 100A is carried out as a result of a computer executing instructions written in a program which is provided in such a manner that the program is stored in a computer readable information recording medium.

As shown in FIG. 4, the measuring apparatus 100A has a walking determining part 110 which processes an output signal from the pedometer 10, to determine whether a to-be-measured person is currently walking. Further, the measuring apparatus 100A has a horizontal moving rate measuring part 130 which processes respective output signals from the walking determining part 110 and the GPS 30, to detect a change in a horizontal position at which the to-be-measured person is thus walking, and measure a moving rate in a horizontal direction.

Further, the measuring apparatus 100A has an output determining part 150A. The output determining part 150A processes respective output signals from the walking determining part 110 and the horizontal moving rate measuring part 130, to determine whether the to-be-measured person is currently going up/down stairs. Then, the output determining part 150A provides an instruction to a time/number of steps difference calculating part 160A such that calculation results of the time/number of steps difference calculating part 160A, described later, are output to the data storing device 200, only when the output determining part 150 determines that the to-be-measured person is currently going up/down stairs.

The time/number of steps difference calculating part 160A which the measuring apparatus 100A also has processes respective output signals from the pedometer 10, the GPS 30, the walking determining part 110 and the output determining part 150A, to calculate a time elapsed during when the to-be-measured person is going up/down stairs and the number of steps during this time. Then, the time/number of steps difference calculating part 160A outputs these calculation results to the data storing device 200.

Further, the measuring apparatus 100A includes a data storing part 170 which is configured to store data used by the time/number of steps difference calculating part 160A for the calculation.

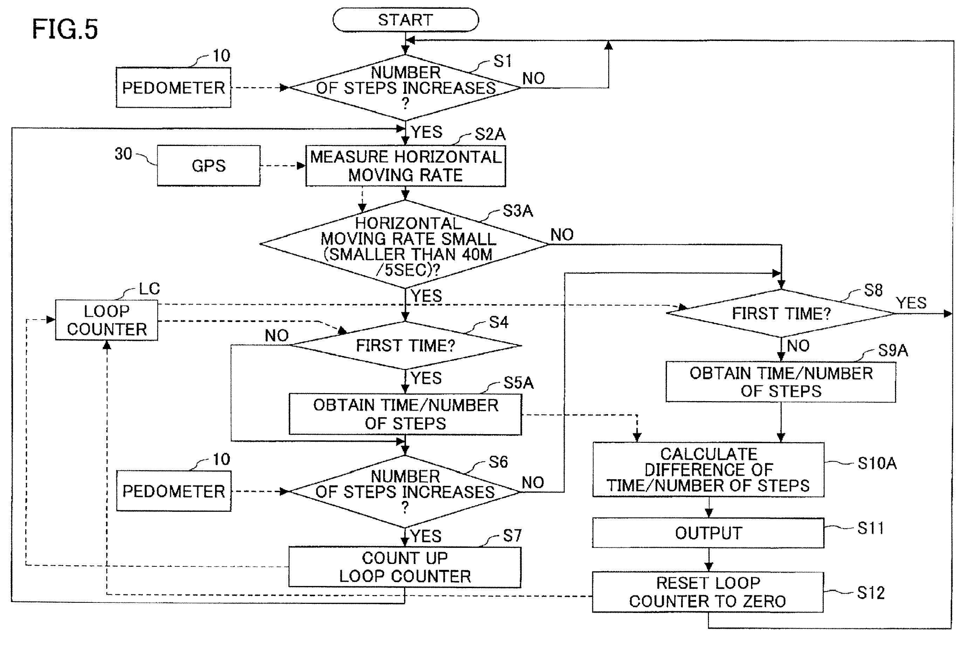

FIG. 5 shows a procedure carried out by the measuring apparatus 100A.

It is noted that, here, it is assumed that a to-be-measured person carries the cellular phone having the measuring apparatus 100A, the pedometer 10 and the GPS 30, provided in the inside thereof.

In FIG. 5, the walking determining part 110 reads an output signal of the pedometer 10 in step S1, and compares the thus-obtained output count value of the number of steps from the pedometer 10 with a previously obtained output count value of the number of steps from the pedometer 10. Then, when the comparison result is that the output count value of the number of steps has increased (YES), step S2A is then carried out. On the other hand, when the comparison result is that the output count value of the number of steps has not increased (NO in step S1), the comparison process of step S1 is repeated at a predetermined interval.

The same as the first embodiment, it is noted that, the pedometer 10 is provided in the inside of the cellular phone, and the to-be-measured person carries the cellular phone. Accordingly, the output signal of the pedometer 10 indicates the number of steps of the to-be-measured person himself or herself. During when the to-be-measured person is currently walking, the output count value of the number of steps of the pedometer 10 gradually increases accordingly. Therefore, the walking determining part 110 detects the increase in the output count value of the number of steps by receiving the output signal of the pedometer 10 which indicates the output count value of the number of steps of the pedometer 10. Thus, the walking determining part 110 can detect a state of the to-be-measured person currently walking.

In step S2A, the horizontal moving rate measuring part 130 receives an output signal of the GPS 30, and obtains a difference value of a horizontal position during a predetermined time, for example, 5 seconds. Thus, the horizontal moving rate measuring part 130 obtains a moving rate in a horizontal direction.

It is noted that, the output signal of the GPS 30 indicates a horizontal position measurement value. When the to-be-required person goes up stairs for example, or goes down stairs, a horizontal position of the to-be-measured person gradually changes accordingly. As a result, the horizontal position measurement value gradually changes accordingly. The horizontal moving rate measuring part 130 receives the output signal indicating the horizontal position measurement value from the GPS 30, and thus, can detect that the horizontal position of the to-be-measured person gradually changes. It is noted that, the operation in step S2A will be described in more detail with reference to FIG. 3.

Then, in step S3A, an absolute value of the moving rate in the horizontal direction thus obtained in step S2A, i.e., an absolute value of the difference in horizontal position measured during 5 seconds is compared with a threshold, for example, 40 meters. In this case, when the horizontal position measured has changed larger than 40 m during 5 seconds, it cannot be determined that the to-be-measured person goes up/down stairs. Thus, it is determined that the to-be-measured person does not go up/down stairs. When the horizontal position measured has changed equal to or smaller than 40 m during 5 seconds, it is determined that the to-be-measured person goes up/down stairs.

Prior to step S3A, it has been determined in step S1 that the to-be-measured person is currently going up/down stairs. Therefore, step S3A is carried out under such an assumption that, when a horizontal moving amount per a predetermined time is relatively small even when the to-be-measured person is currently walking, it is reasonable to rather determine that the to-be-measured person goes up/down stairs than determining that the to-be-measured person walks on a horizontal plane.

Further, it is noted that, before reaching step S3A, it is determined whether the to-be-measured person is currently walking in step S1. Assuming that, for example, the to-be-measured person is currently taking an elevator, an escalator or such, and is not currently walking, it is determined in step S1 that the to-be-measured person is not currently walking. Therefore, in this case, step S3A is not reached. As a result, an erroneous determination that the to-be-measured person is currently going up/down stairs can be positively avoided in such a case.

When the comparison result in step S3A is YES, i.e., when the absolute value of the moving rate in the horizontal direction is equal to or smaller than "40m/5s" (i.e., 40 meters per 5 seconds), it is determined that the to-be-measured person is currently going up/down stairs, i.e., the to-be-measured person is currently going up/down stairs.

That is, when it is determined in step S1 that the to-be-measured person is currently walking, and also, it is determined in step S3A that the to-be-measured person is currently going up/down stairs, it is finally determined that the to-be-measured person is currently going up/down stairs.

When the comparison result in step S3A is NO, i.e., when the absolute value of the moving rate in the horizontal direction is larger than "40m/5s" (i.e., 40 meters per 5 seconds), it is determined that the moving rate in the horizontal direction exceeds such a value as to determine that the to-be-measured person is currently going up/down stairs. As a result, step S8 is carried out then. Operation in step S8 will be described later.

When the comparison result in step S3A is YES, step S4 is carried out then. In step S4, first, it is determined whether execution of step S4 is for the first time. In order to determine how many times S4 step has been executed, a loop counter LC is provided in the measuring apparatus 100A. The loop counter LC is incremented by one in step S7 described later. The loop counter LC is reset to zero in step S12 described later each time a finish of the to-be-measured person's going up/down stairs is detected.

Also in the flow of FIG. 5, it is noted that, after it is determined that the to-be-measured person starts going up/down stairs as a result of the comparison result of step S1 being YES and also the comparison result of step S3A being YES, loop operation of steps S2A, S3A, S4, SSA, S6, S7 and then again S2A is repeated until the comparison result of step S1 or the comparison result of step S2 becomes NO. The loop counter LC is incremented by one in step S7 each time the loop operation is executed. Therefore, the count value of the loop counter LC indicates the number of times of repetition of the loop operation. Further, when it is determined that the to-be-measured person's going up/down stairs has finished, the count value of the loop counter LC is reset to zero in step S12. Therefore, the count value of the loop counter LC indicates the number of times of repetition of the loop operation since when the to-be-measured person has started going up/down stairs.

In step S4, it is determined whether step S4 is currently carried out for the first time. When the determination result of step S4 is YES, i.e., when the count value of the loop counter LC is zero, this means that the current loop operation is of the first time, and thus, this means that the to-be-measured person currently has started going up/down stairs.

In this case, step S5A is carried out in which a current time and the current output count value of the number of steps indicated by the output signal of the pedometer 10 are stored in the data storing part 170 as values at a start of going up/down stairs. Information of the current time is obtained from a digital clock of the cellular phone provided with the measuring apparatus 100A.

When the determination result of step S4 is NO, this means that the current execution of step S4 is of the second time or more, and thus, this means that the values at the start of going up/down stairs have been already stored in the data storing part 170 in the loop operation of the first time. In this case, step S5A is skipped, and then, step S6 is carried out.

In step S6, the output count value of the number of steps indicated by the output signal of the pedometer 10 is read again. Then, the thus-read output count value of the number of steps is compared with the output value of the number of steps previously read. This output value of the number of steps previously read to be compared there is, for the loop operation of the first time, the output count value of the number of steps obtained in step S1. The output value of the number of steps previously read to be compared there is, for the loop operation of the second time or more, the output count value of the number of steps obtained in step S6 of the previous time of loop operation.

When the comparison result of step S6 is that the output value of the number of steps currently obtained is larger than the output value of the number of steps previously obtained (YES), i.e., that the output value of the number of steps has increased, step S7 is carried out then. In step S7, the loop counter LC is incremented by one, and then, from step S2A, the above-mentioned loop operation is repeated.

When the comparison result of step S6 is NO, i.e., the output value of the number of steps currently obtained is not lager than the output value of the number of steps previously obtained, this means that the number of steps has not increased, and thus, it is determined that the to-be-measured person has finished going up/down stairs, or when the comparison result of step S3A is NO in the loop operation of the second time or more, i.e., the absolute value of the moving rate in the horizontal direction is larger than 40m/5s, it is determined that the to-be-measured person has finished going up/down stairs.

In step S6, the output count value of the number of steps is compared between the current time and the previous time. During this time, a change amount (i.e., difference value) of altitude is measured for 5 seconds in step S3A. This means that, at least 5 seconds have elapsed from the time at which the output count value of the number of steps read previously. When the comparison result of step S6 is NO, this means that the output count value of the number of steps has not increased during the 5 seconds. That is, during 5 seconds, the to-be-measured person has not walked any step. In such a case, it is determined that the to-be-measured person has finished walking, i.e., has finished going up/down stairs.

When the comparison result of step S3A in the loop operation of the second time or more is NO, it is determined that the current moving rate in the horizontal direction exceeds such a value as to determine that the to-be-measured person is currently going up/down stairs. As a result, it is determined that the to-be-measured person's going up/down stairs has been finished. In this case, since it is determined in the previous step S6 that the output value of the number of steps has increased, it is determined that the to-be-measured person has still continued walking, with finishing going up/down stairs, and then, instead, with starting walking on a horizontal plane.

When thus the comparison result of step S3A or S6 is NO, i.e., it is determined that the to-be-measured person is not currently going up/down stairs or that the to-be-measured person has finished going up/down stairs, the count value of the loop counter LC is read in step S8. When the count value of the loop counter LC is zero in step S8, the current loop operation is of the first time. In this case, step S1 is then carried out. In step S1, it is determined whether the output count value of the number of steps has increased or not.

Next, when the determination result of step S8 is NO, respective values of time/number of steps are obtained again as values at a finish of going up/down stairs, in step S9A. That is, the same as in step S5 mentioned above, the current time and the current output count value of the number of steps indicated by the output signal of the pedometer 10 are stored as the values at a finish of going up/down stairs in the data storing part 170.

Next, in step S10A, differences between the respective values of time/number of steps at the start of going up/down stairs obtained in step S5 and the respective values of time/number of steps of at the finish of going up/down stairs obtained in step S9A are calculated. The thus-obtained difference values are output to the data storing device 200 in step S11. After that, the loop counter LC is reset to zero (step S12), and the current process is finished. After that, step S1 is again carried out, and then, the above-mentioned process is repeated.

When the determination result of step S8 is YES, this means that the current loop operation is of the first time, the process of step S1 is carried out again.

When step S8 is carried out directly after step S3A in the loop operation of the first time, this means that no going up/down stairs has been started. In this case, no values of time/number of steps have been obtained. Therefore, in this case, there is no meaning to carry out step S9A of obtaining respective values of time/number of steps at a finish of going up/down stairs. Therefore, in this case, the loop counter LC is read in step S8, which results in YES in this case since it is the loop operation of the first time, and step S1 is then carried out without carrying out step S9A. Further, a situation that, in the loop operation of the first time, step S8 is carried out directly after step S6, is a situation that this is immediately after respective values of time/number of steps have been obtained in step S5A. Even if respective values of time/number of steps are obtained in step S9A in such a situation, respective difference values of time/number of steps then calculated in step S10A become approximately zeros accordingly. Therefore, also in this case, the loop counter LC is read in step S8, which results in YES in this case since it is the loop operation of the first time, and step S1 is then carried out without carrying out step S9A.

Next, with reference to FIG. 3, a horizontal moving rate measuring process (step S2A) of the flow of FIG. 5 will be described in detail.

First, in step S21, horizontal position measurement for the first time is carried out.

After that, in step S22, time elapse is determined. That is, when a predetermined time, in this embodiment, 5 seconds, has elapsed, a next step S23 is carried out.

In step S23, horizontal position measurement for the second time is carried out.

In step S24, a difference between a horizontal position measurement value obtained in step S21 and a horizontal position measurement value obtained in step S23, as the moving rate in the horizontal direction of the to-be-measured person, is calculated.

Thus, the moving rate in the horizontal direction of the to-be-measured person, i.e., a moving amount during 5 seconds is obtained.

Next, a third embodiment according to the present invention will be described.

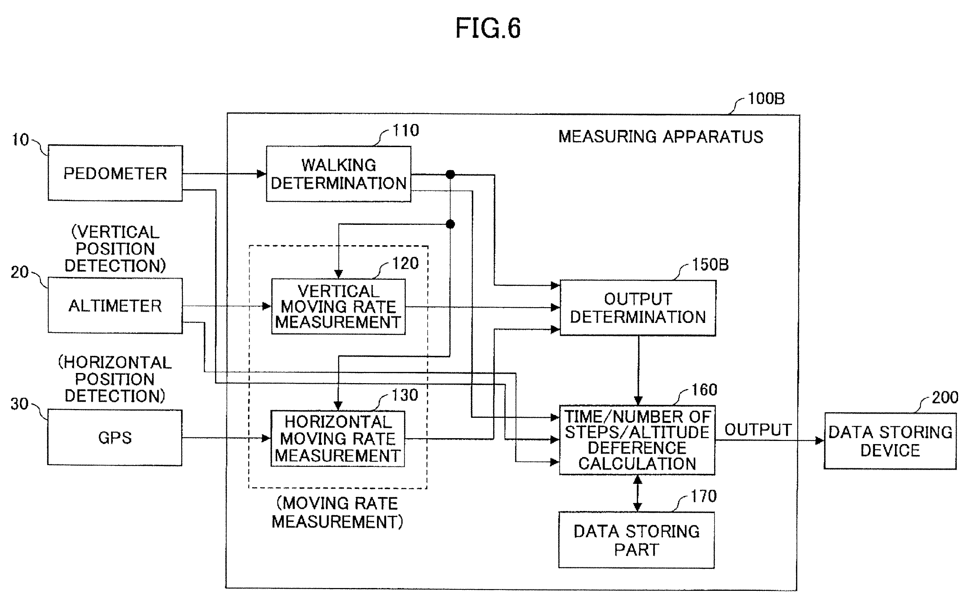

FIG. 6 shows a block diagram of a measuring apparatus 100B in the third embodiment of the present invention.

The same as the measuring apparatus 100 in the first embodiment, the measuring apparatus 100B in the embodiment is used in such a manner that it is provided in the inside of a cellular phone. In this case, in the inside of the cellular phone, as shown in FIG. 6, also a pedometer 10 configured to measure the number of steps of a to-be-measured person, an altimeter configured to measure an altitude at which the measuring apparatus 100B is located and a GPS 30 configured to measure a horizontal position at which the measuring apparatus 100B is located, and a data storing device 200, are provided.

Further, as will be described later with reference to FIG. 9, operation carried out by the measuring apparatus 100B is carried out as a result of a computer executing instructions written in a program which is provided in such a manner that the program is stored in a computer readable information recording medium.

As shown in FIG. 6, the measuring apparatus 100B has a walking determining part 110 which processes an output signal from the pedometer 10, to determine whether a to-be-measured person is currently walking. Further, the measuring apparatus 100B has a vertical moving rate measuring part 120 which processes respective output signals from the walking determining part 110 and the altimeter 20, to detect a change in a vertical position or an altitude at which the to-be-measured person is thus walking, and measure a moving rate in a vertical direction. Further, the measuring apparatus 100B has a horizontal moving rate measuring part 130 which processes respective output signals from the walking determining part 110 and the GPS 30, to detect a change in a horizontal position at which the to-be-measured person is thus walking, and measure a moving rate in a horizontal direction.

Further, the measuring apparatus 100B has an output determining part 150B. The output determining part 150B processes respective output signals from the walking determining part 110, the vertical moving rate measuring part 120 and the horizontal moving rate measuring part 130, to determine whether the to-be-measured person is currently going up/down stairs. Then, the output determining part 150B provides an instruction to a time/number of steps/altitude difference calculating part 160 such that calculation results of the time/number of steps/altitude difference calculating part 160, described later, are to be output to the data storing device 200, only when the output determining part 150B determines that the to-be-measured person is currently going up/down stairs.

The time/number of steps/altitude difference calculating part 160 which the measuring apparatus 100B also has processes respective output signals from the pedometer 10, the altimeter 20 and the GPS 30, the walking determining part 110 and the output determining part 150B, to calculate a time elapsed during when the to-be-measured person is going up/down stairs, the number of steps during this time and the altitude difference during the time. Then, the time/number of steps/altitude difference calculating part 160 outputs these calculation results to the data storing device 200.

Further, the measuring apparatus 100B includes a data storing part 170 which is configured to store data used by the time/number of steps/altitude difference calculating part 160 for the calculation.

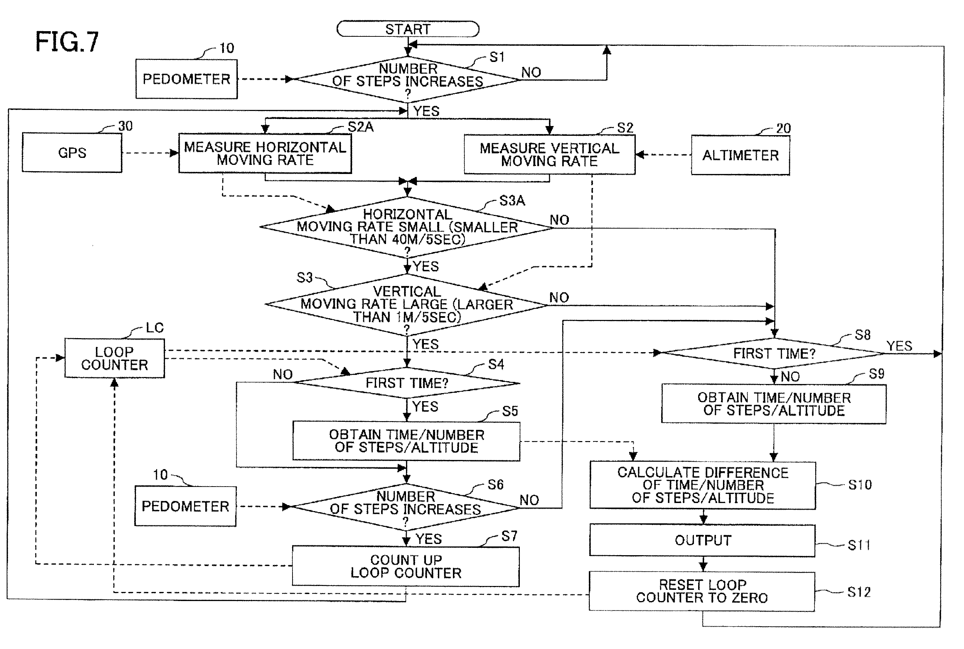

FIG. 7 shows a procedure carried out by the measuring apparatus 100B.

It is noted that, here, it is assumed that a to-be-measured person carries a cellular phone having the measuring apparatus 100B, the pedometer 10, the altimeter 20 and the GPS 30, provided in the inside thereof.

In FIG. 7, the walking determining part 110 reads an output signal of the pedometer 10 in step S1, and compares the thus-obtained output count value of the number of steps from the pedometer 10 with a previously obtained output count value of the number of steps from the pedometer 10. Then, when the comparison result is that the output count value of the number of steps has increased (YES), step S2 and step S2A are then carried out in parallel. On the other hand, when the comparison result is that the output count value of the number of steps has not increased (NO in step S1), the comparison process of step S1 is repeated at a predetermined interval.

The same as the first embodiment, it is noted that, the pedometer 10 is provided in the inside of the cellular phone, and the to-be-measured person carries the cellular phone. Accordingly, the output signal of the pedometer 10 indicates the number of steps of the to-be-measured person himself or herself. During when the to-be-measured person is currently walking, the output count value of the number of steps of the pedometer 10 gradually increases accordingly. Therefore, the walking determine part 110 detects the increase in the output count value of the number of steps by receiving the output signal of the pedometer 10 which indicates the output count value of the number of steps of the pedometer 10. Thus, the walking determining part 110 can detect a state of the to-be-measured person currently walking.

In step S2, the vertical moving rate measuring part 120 receives an output signal of the altimeter 20, and obtains a difference value of altitude during a predetermined time, for example, 5 seconds, i.e., a moving rate in the vertical direction.

It is noted that, the output signal of the altimeter 20 indicates an altitude measurement value. When the to-be-required person goes up stairs for example, or goes down stairs, altitude of the to-be-measured person gradually increases or decreases accordingly. As a result, the altitude measurement value gradually increases or decreases accordingly. The vertical moving rate measuring part 120 receives the output signal indicating the altitude measurement value from the altimeter 30, and thus, can detect that the altitude of the to-be-measured person gradually increases or decreases. It is noted that, the operation in step S2 will be described later in more detail with reference to FIG. 3.

In step S2A, which is carried out in parallel to step S2, the horizontal moving rate measuring part 130 receives an output signal of the GPS 30, and obtains a difference value of a horizontal position during a predetermined time, for example, 5 seconds. Thus, the horizontal moving rate measuring part 130 obtains a moving rate in a horizontal direction.

It is noted that, the output signal of the GPS 30 indicates a horizontal position measurement value. When the to-be-required person goes up stairs for example, or goes down stairs, a horizontal position of the to-be-measured person gradually changes accordingly. As a result, the horizontal position measurement value gradually changes accordingly. The horizontal moving rate measuring part 130 receives the output signal indicating the horizontal position measurement value from the GPS 30, and thus, can detect that the horizontal position of the to-be-measured person gradually changes. It is noted that, the operation in step S2A will be described later in more detail with reference to FIG. 3.

Then, in step S3A, an absolute value of the moving rate in the horizontal direction thus obtained in step S2A, i.e., an absolute value of the difference in horizontal position measured during 5 seconds is compared with a threshold, for example, 40 meters. In this case, when the horizontal position measured has changed larger than 40 m during 5 seconds, it cannot be determined that the to-be-measured person goes up/down stairs. Thus, it is determined that the to-be-measured person does not go up/down stairs. When the horizontal position measured changed equal to or smaller than 40 m during 5 seconds, it is determined that the to-be-measured person goes up/down stairs.

Prior to step S3A, it has been determined in step S1 that the to-be-measured person is currently going up/down stairs. Therefore, step S3A is carried out under such an assumption that, when a horizontal moving amount per a predetermined time is relatively small even when the to-be-measured person is currently walking, it is reasonable to determine that the to-be-measured person goes up/down stairs rather than determining that the to-be-measured person walks on a horizontal plane.

Further, it is noted that, before reaching step S3A, it is determined whether the to-be-measured person is currently walking in step S1. Assuming that, for example, the to-be-measured person is currently taking an elevator, an escalator or such, and is not actually walking, it is determined in step S1 that the to-be-measured person is not currently walking. Therefore, in this case, step S3A is not reached. As a result, an erroneous determination that the to-be-measured person is currently going up/down stairs can be positively avoided.

When the comparison result in step S3A is YES, i.e., when the absolute value of the moving rate in the horizontal direction is equal to or smaller than "40m/5s" (i.e., 40 meters per 5 seconds), it is determined that the to-be-measured person is currently going up/down stairs.

That is, when it is determined in step S1 that the to-be-measured person is currently walking, and also, it is determined in step S3A that the to-be-measured person is currently going up/down, it is then determined that the to-be-measured person is currently going up/down stairs.

When the comparison result in step S3A is NO, i.e., when the absolute value of the moving rate in the horizontal direction is larger than "40m/5s" (i.e., 40 meters per 5 seconds), it is determined that the moving rate in the horizontal direction exceeds such a value as to determine that the to-be-measured person is currently going up/down stairs. As a result, step S8 is carried out then. Operation in step S8 will be described later.

When the comparison result in step S3A YES, i.e., when the moving rate in the horizontal direction falls within a moving rate in the horizontal direction such as to determine that the to-be-measured person goes up/down stairs, step S3 is carried out then.

In step S3, an absolute value of the moving rate in the vertical direction obtained in step S2 as mentioned above, i.e., an absolute value of the difference in altitude measured during 5 seconds is compared with a threshold, for example, 1 meter. In this case, when the altitude measured has increased equal to or larger than 1 m during 5 seconds, it is determined that the to-be-measured person goes up stairs. When the altitude measured decreases larger than 1 m during 5 seconds, it is determined that the to-be-measured person goes down stairs.

It is noted that, before reaching step S3, it is determined whether the to-be-measured person is currently walking in step S1. Assuming that, for example, the to-be-measured person is currently taking an elevator, an escalator or such, and is not actually walking, the altitude of the to-be-measured person changes. However, in such a case, it is determined in step S1 that the to-be-measured person is not currently walking. Therefore, in this case, step S3 is not reached. As a result, an erroneous determination that the to-be-measured person is currently going up/down stairs can be positively avoided.

When the comparison result of step S3 is YES, that is, when the absolute value of the moving rate in the vertical direction of the to-be-measured person is equal to or larger than 1m/5s, it is finally determined that the to-be-measured person is currently going up/down stairs.

On the other hand, when the comparison result of step S3 is NO, that is, when the absolute valued of the moving rate in the vertical direction of the to-be-measured person is smaller than 1m/5s, it is determined that the to-be-measured person does not move in the vertical direction so far as to be determined as going up/down stairs, and step S8 is carried out. Processes after being carried out from step S8 will be described later.

When the comparison result of step S3 is YES, step S4 is carried out. In step S4, first, it is determined whether execution of step S4 is the for first time. In order to determine how many times S4 step has been executed, a loop counter LC is provided in the measuring apparatus 100B. The loop counter LC is incremented by one in step S7 described later. The loop counter LC is reset to zero in step S12 described later each time a finish of the to-be-measured person's going up/down stairs is detected.

It is noted that, after it is determined that the to-be-measured person starts going up/down stairs as a result of the comparison result of step S1 being YES, the comparison result of step S3A being YES, and also the comparison result of step S3 being YES, loop operation of steps S2A, S2, S3A, S3, S4, S5A, S6, S7 and then again S2A is repeated until the comparison result of step S1, the comparison result of step S3A becomes NO, or the comparison result of step S3 becomes NO. The loop counter LC is incremented by one in step S7 each time the loop operation is executed. Therefore, the count value of the loop counter LC indicates the number of times of repetition of the loop operation. Further, when it is determined that the to-be-measured person's going up/down stairs has finished, the count value of the loop counter LC is reset to zero in step S12. Therefore, the count value of the loop counter LC indicates the number of times of repetition of the loop operation since when the to-be-measured person has started going up/down stairs.

In step S4, it is determined whether step S4 is currently carried out for the first time. When the determination result of step S4 is YES, i.e., when the count value of the loop counter LC is zero, this means that the current loop operation is of the first time, and thus, this means that the to-be-measured person currently has started going up/down stairs.

In this case, step S5 is carried out in which a current time, the current output count values of the number of steps indicated by the output signal of the pedometer 10 and the altitude measurement value indicated by the altimeter 20 are stored in the data storing part 170 as values at a start of going up/down stairs. Information of the current time is obtained from a digital clock of the cellular phone provided with the measuring apparatus 100B.

When the determination result of step S4 is NO, this means that the current execution of step S4 is for the second time or more, and thus, this means that the values at the start of going up/down stairs have been already stored in the data storing part 170 in the loop operation of the first time. In this case, step S5 is skipped, and then, step S6 is carried out.

In step S6, the output count value of the number of steps indicated by the output signal of the pedometer 10 is read again. Then, the thus-read output count value of the number of steps is compared with the output value of the number of steps previously read. This output value of the number of steps previously read to be compared there is, for the loop operation of the first time, the output count value of the number of steps obtained in step S1. The output value of the number of steps previously read to be compared there is, for the loop operation of the second time or more, the output count value of the number of steps obtained in step S6 of the previous time of loop operation.

When the comparison result of step S6 is that the output value of the number of steps currently obtained is larger than the output value of the number of steps previously obtained (YES), i.e., that the output value of the number of steps has increased, step S7 is carried out then. In step S7, the loop counter LC is incremented by one, and then, from steps S2 and S2A, the above-mentioned loop operation is repeated.

When the comparison result of step S6 is NO, i.e., the output value of the number of steps currently obtained is not lager than the output value of the number of steps previously obtained, this means that the number of steps has not increased, and thus, it is determined that the to-be-measured person has finished going up/down stairs, or when the comparison result of step S3A is NO in the loop operation of the second time or more, i.e., the absolute value of the moving rate in the horizontal direction is larger than 40m/5s, further or when the comparison result of step S3 is NO in the loop operation of the second time or more, i.e., the absolute value of the moving rate in the vertical direction is smaller than 1m/5s, it is determined that the to-be-measured person has finished going up/down stairs.

In step S6, the output count value of the number of steps is compared between the current time and the previous time. During this time, a change amount (i.e., difference value) of horizontal position and altitude are measured for each 5 seconds in steps S3A and S3. This means that, at least 10 seconds have elapsed from the time at which the output count value of the number of steps read previously. When the comparison result of step S6 is NO, this means that the output count value of the number of steps has not increased during the 10 seconds. That is, during 10 seconds, the to-be-measured person has not walked any step. In such a case, it is determined that the to-be-measured person has finished walking, i.e., has finished going up/down stairs.

When the comparison result of step S3A or S3 in the loop operation of the second time or more is NO, it is determined that the current moving rate in the horizontal direction exceeds such a value as to determine that the to-be-measured person is currently going up/down stairs or that the current moving rate in the vertical direction does not amount to such a value as to determine that the to-be-measured person is currently going up/down stairs. As a result, it is determined that the to-be-measured person's going up/down stairs has been finished. In this case, since it is determined in the previous step S6 that the output value of the number of steps has increased, it is determined that the to-be-measured person has still continued walking, with finishing going up/down stairs, and then, instead, with starting walking on a horizontal plane.

When thus the comparison result of step S3A, S3 or S6 is NO, i.e., it is determined that the to-be-measured person is not currently going up/down stairs or that the to-be-measured person has finished going up/down stairs, the count value of the loop counter LC is read in step S8. When the count value of the loop counter LC is zero in step S8, the current loop operation is of the first time. In this case, step S1 is then carried out. In step S1, it is determined whether the output count value of the number of steps has increased or not.

Next, when the determination result of step S8 is NO, respective values of time/number of steps/altitude are obtained again as values at a time of a finish of going up/down stairs, in step S9. That is, the same as in step S5 mentioned above, the current time, the current output count value of the number of steps indicated by the output signal of the pedometer 10, and the altitude measurement value indicated by the altimeter 20 are stored as the values at a finish of going up/down stairs in the data storing part 170.

Next, in step S10, differences between the respective values of time/number of steps/altitude of the start of going up/down stairs obtained in step S5 and the respective values of time/number of steps/altitude of the finish of going up/down stairs obtained in step S9 are calculated. The thus-obtained difference values are output to the data storing device 200 in step S11. After that, the loop counter LC is reset to zero (step S12), and the current process is finished. After that, step S1 is again carried out, and then, the above-mentioned process is repeated.

Next, with reference to FIG. 3, a horizontal moving rate measuring process (step S2A) of the flow of FIG. 7 will be described in detail.

First, in step S21, horizontal position measurement for the first time is carried out.

After that, in step S22, time elapse is determined. That is, when a predetermined time, in this embodiment, 5 seconds, has elapsed, a next step S23 is carried out.

In step S23, horizontal position measurement for the second time is carried out.

In step S24, a difference between a horizontal position measurement value obtained in step S21 and a horizontal position measurement value obtained in step S23, as the moving rate in the horizontal direction of the to-be-measured person, is calculated.

Thus, the moving rate in the horizontal direction of the to-be-measured person, i.e., a moving amount during 5 seconds is obtained.

Next, with reference to FIG. 3, a vertical moving rate measuring process (step S2) of the flow of FIG. 7 will be described in detail.

First, in step S21, vertical position measurement for the first time is carried out.

After that, in step S22, time elapse is determined. That is, when a predetermined time, in this embodiment, 5 seconds, has elapsed, a next step S23 is carried out.

In step S23, vertical position measurement for the second time is carried out.

In step S24, a difference between a vertical position measurement value obtained in step S21 and a vertical position measurement value obtained in step S23, as the moving rate in the vertical direction of the to-be-measured person, is calculated.

Thus, the moving rate in the vertical direction of the to-be-measured person, i.e., a moving amount during 5 seconds is obtained.

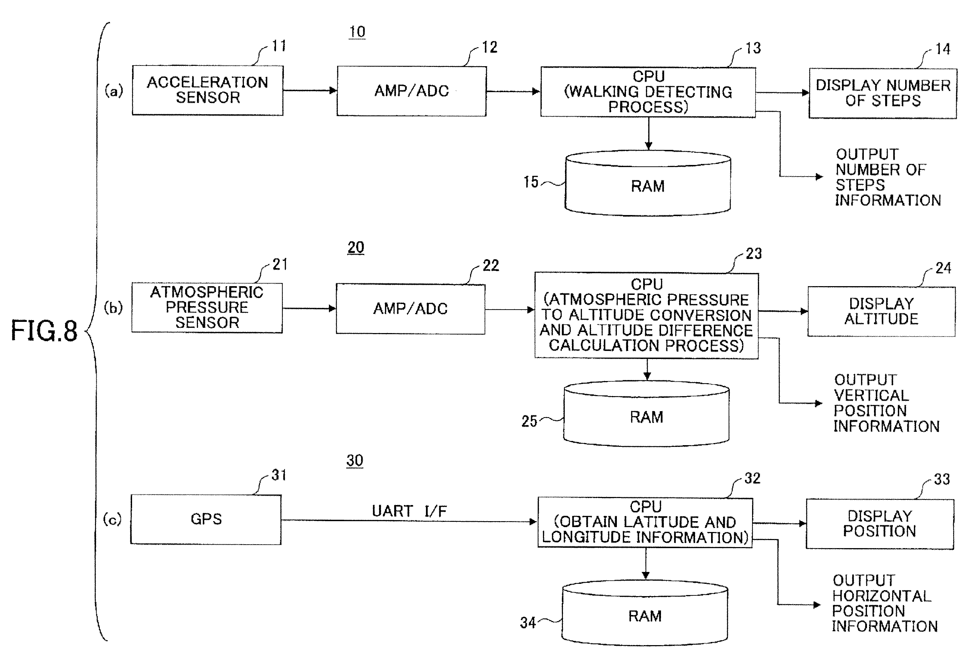

Next, with reference to FIG. 8, respective configuration examples of the pedometer 10, the altimeter 20 and the GPS 30 used in each embodiment mentioned above will be described.

FIG. 8, (a), shows a configuration example of the pedometer 10.

As shown in FIG. 8, (a), the pedometer 10 includes an acceleration sensor 11, an amplifier and A-D converter 12, a CPU 13, a RAM 15 and a number of steps display device 14.

In this configuration, acceleration of a to-be-measured person is measured by the acceleration sensor 11, a measurement signal of the acceleration sensor 11 is then amplified and is converted into a digital signal by the amplifier and A-D converter 12, and after that, is used by the CPU 13 for calculation. The CPU 13 uses the RAM 15 as a temporary storage device in the calculation. By the calculation of the CPU 13, walking operation of the to-be-measured person is detected and also, a count value of the number of steps is obtained. The count value of the number of steps is displayed on the number of steps display device 14, and also, an output signal indicating the count value of the number of steps is provided to the measuring apparatus 100, 100A or 100B.

FIG. 8, (b), shows a configuration example of the altimeter 20.

As shown in FIG. 8, (b), the altimeter 20 includes an atmospheric pressure sensor 21, an amplifier and A-D converter 22, a CPU 23, a RAM 25 and an altitude display device 24.

In this configuration, altitude of a to-be-measured person is measured by the atmospheric pressure sensor 21, a measurement signal of the atmospheric pressure sensor 21 is then amplified and is converted into a digital signal by the amplifier and A-D converter 22, and after that, is used by the CPU 23 for calculation. The CPU 23 uses the RAM 25 as a temporary storage device in the calculation. By the calculation of the CPU 23, an altitude measurement value for the to-be-measured person is obtained. The altitude measurement value is displayed on the altitude display device 24, and also, an output signal indicating the altitude measurement value is provided to the measuring apparatus 100, 100A or 100B.

FIG. 8, (c), shows a configuration example of the GPS 30.

As shown in FIG. 8, (c), the GPS 30 includes a GPS unit 31, a CPU 32, a RAM 34 and a position display device 33.

In this configuration, the GPS unit 31 receives radio waves from a plurality of satellites, converts them into digital signals, and provides them to the CPU 32. The CPU 32 carries out calculation on information obtained from the digital signals. The CPU 32 uses the RAM 34 as a temporary storage device in the calculation. By the calculation of the CPU 32, horizontal position information for the to-be-measured person is obtained. The horizontal position information is displayed on the position display device 33, and also, an output signal indicating the horizontal position information is provided to the measuring apparatus 100, 100A or 100B.

FIG. 9 shows a block diagram of a computer for illustrating a case where the computer is used to carry out information processing in the measuring apparatus 100, 100A or 100B in each embodiment.

As shown in FIG. 9, the computer 500 includes a CPU 501, an operation device 502, a display device 503, a memory 504, a hard disk drive 505, a CD-ROM drive 506 and a modem 508. The CPU 501 carries out various sorts of operation by executing instructions written in a given program. The operation device 502 includes a keyboard, a mouse, and so forth, and is used for a user to input operation contents and data. The display device 503 is a CRT, a liquid crystal display device or such, and is used to display, to a user, a processing result and so forth,. The memory 504 is used to store a program executed by the CPU 504, data and so forth, and also, is used as a working area. The hard disk drive 505 is used to store a program, data and so forth. The CD-ROM drive 506 is used to load a program externally, or load data with the use of a CD-ROM 507 used as a computer readable information recording medium. The modem 508 is used to download a program from an external server via a communication network 509 such as the Internet, a LAN or such.

Further, as shown in FIG. 9, the computer 500 may have some or all of the pedometer 10, the altimeter 20 and the GPS 30 connected thereto.

The computer 500 loads or downloads a program, for causing the CPU 501 to carry out operation which the measuring apparatus 100, 100A or 100B carries out, by means of the CD-ROM 507, or by means of the communication network 509. Then, the program is installed in the hard disk drive 505, is loaded on the memory, and is executed by the CPU 501. As a result, the computer 500 carries out operation of the measuring apparatus 100, 100A or 100B.

It is noted that, in a case where the measuring apparatus 100, 100A or 100B is provided in the inside of a cellular phone, the hared disk drive 505 and the CD-ROM drive 506 may be replaced by an SD card drive, a mini SD card drive or such.

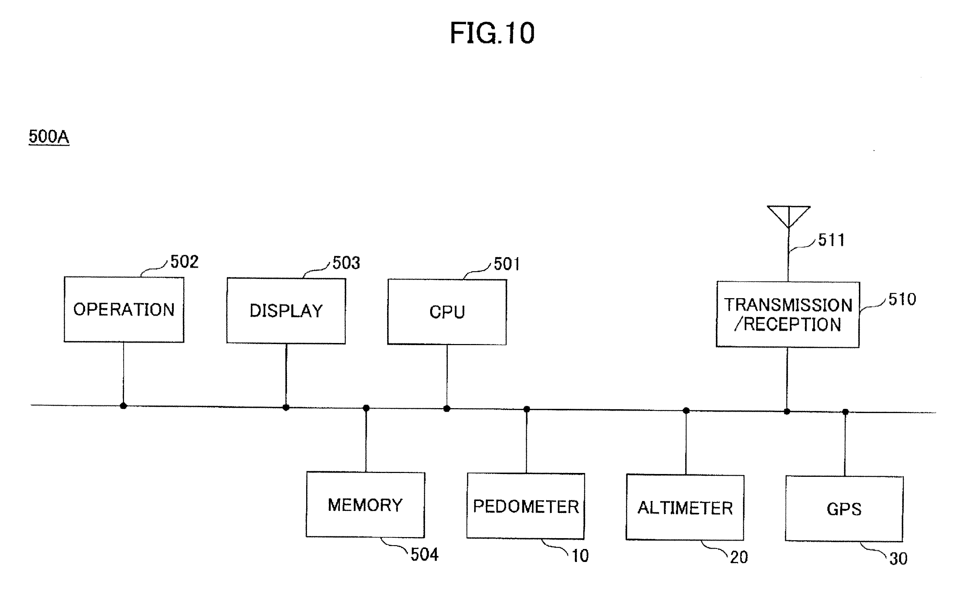

FIG. 10 shows a block diagram of a cellular phone for illustrating a case where the cellular phone has a configuration to carry out information processing in the measuring apparatus 100, 100A or 100B in each embodiment.

As shown in FIG. 10, the cellular phone 500A includes a CPU 501, a key operation device 502, a screen display device 503, a memory 504 and a transmission/reception unit 510. The CPU 501 carries out various sorts of operation such as operation concerning radio telephone conversation, electronic mail transmission/reception, or such, by executing instructions written in a given program. The key operation device 502 includes a keyboard or such, and is used for a user to carry out operation concerning radio telephone conversation, electronic mail transmission/reception or such. The screen display device 503 is used to display, to a user, message contents of an electronic mail or such. The memory 504 is a ROM, a RAM, an SD card, a mini SD card or such, is used to store a program executed by the CPU 504, data and so forth, and also, is used as a working area. The transmission/reception unit 510 carries out signal processing concerning radio telephone conversation, or electronic mail transmission/reception with the use of a radio communication circuit via an antenna 511.

Further, as shown in FIG. 10, the cellular phone 500A may have some or all of the pedometer 10, the altimeter 20 and the GPS 30, provided in the inside thereof, or externally connected thereto.

In the cellular phone 500A, a program for causing the CPU 501 to carry out operation which the measuring apparatus 100, 100A or 100B carries out is mounted in the memory 504, and is executed by the CPU 501. As a result, the cellular phone 500A carries out operation of the measuring apparatus 100, 100A or 100B.

Further, on the screen display device 503, measurement information, which is determined to be output, by the above-mentioned output determining part 150, 150A or 150B described above with reference to FIG. 1, 4 or 6, is displayed. A user reads the thus-displayed measurement information and can appropriately understand his or her exercise amount expressed as the measurement information.

A reason why a state of going up/down stairs of a to-be-measured person is detected, and respective sorts of data such as the number of steps, time, altitude difference, horizontal position difference or such, is obtained based on the thus-detected the state of going up/down stairs, in the measuring apparatus in each embodiment of the present invention described above, will now be described.
a) Especially, when a to-be-measured person goes up stairs, an exercise load is applied to the to-be-measured person. Therefore, when a to-be-measured person goes up stairs, it is determined that an effective exercise is carried out, and then, the number of steps and so forth in this case are recorded.
b) However, when a to-be-measured person uses an elevator or such, it can be said that the to-be-measured person does not particularly do exercise. Thus, only from detecting an altitude difference, an actual state of the to-be-measured person cannot be appropriately determined, i.e., whether the to-be-measured person is currently doing effective exercise cannot be determined.
c) Therefore, by combining with detecting an actual walking state of a to-be-required person, whether the to-be-measured person currently goes up/down stairs is determined.

Further, objects of detecting a to-be-measured person's going up/down stairs with the use of the measuring apparatus in each embodiment of the present invention mentioned above will now be described.

Currently, knowledge of metabolic syndrome generally spreads, and along therewith, awareness of avoiding or well treating the metabolic syndrome improves simultaneously, thanks to approach by Health, Labor and Welfare Ministry, influence of medium such as a TV program and so forth. In such an environment, they say that exercise therapy by walking is an answer, which can be most easily and effectively carried out.

When exercise therapy by walking is carried out, they say, it is effective that, by ensuring exercise intensity, i.e., increasing a heart rate to a predetermined level by increasing a speed of walking, body fat is burned in a zone of aerobic exercise. For this purpose, such a walking route is required as to ensure a long walking distance and safety. By introducing going up/down stairs into walking exercise, it is possible to reduce a required walking distance and also, it is possible to reduce a risk in walking through a such a location as a car busy street.

Walking thus introducing going up/down stairs can be carried out at various places such as a school, a company, a park, a shrine, a house, and so forth. There, by carrying out exercise utilizing potential energy and body action of going up/down stairs, it is possible to effectively avoid metabolic syndrome.

The measuring apparatus of each embodiment mentioned above determines whether a to-be-measured person goes up/down stairs, to determine whether a measurement result is to be actually output. Thus, an exercise amount of the to-be-measured person's going up/down stairs is obtained, and therefore, it is possible to appropriately obtain the to-be-measured person's exercise amount even in walking introducing going up/down stairs.

While the invention herein disclosed has been described by means of specific embodiments and applications thereof, numerous modifications and variations could be made thereto by those skilled in the art without departing from the scope of the invention set forth in the claims.

The present application is based on Japanese Priority Application No. 2007-184579 filed July 13, 2007, the entire contents of which are hereby incorporated herein by reference.

## Claims

1. A measuring apparatus comprising:
a walking determining part configured to determine a walking state of a to-be-measured person;
a moving state determining part configured to determine a moving state in a specific direction of the to-be-measured person;
a measurement result output determining part configured to determine whether or not measurement information concerning a movement of the to-be-measured person is to be output, based on a determination result of the walking state of the to-be-measured person by said walking determining part and a determination result of the moving state of the to-be-measured person by the moving state determining part.

2. The measuring apparatus as claimed in claim 1, wherein:
said walking determining part determines the walking state of the to-be-measured person based on an increase in the number of steps within a predetermined time.

3. The measuring apparatus as claimed in claim 1, wherein:
said moving state determining part detects a movement of the to-be-measured person in a vertical direction, and determines a moving state of the to-be-measured person based on a moving amount in the vertical direction within a predetermined time.

4. The measuring apparatus as claimed in claim 3, wherein:
said measurement result output determining part determines that the measurement information concerning a movement of the to-be-measured person is to be output, when said walking determining part determines that the to-be-measured person is currently walking and also said moving state determining part determines that a moving rate of the to-be-measured person in the vertical direction is larger than a predetermined value.

5. The measuring apparatus as claimed in claim 1, wherein:
said measurement result output determining part detects a movement of the to-be-measured person in a horizontal direction, and determines a moving state of the to-be-measured person based on a moving amount in the horizontal direction within a predetermined time.

6. The measuring apparatus as claimed in claim 5, wherein:
said measurement result output determining part determines that the measurement information concerning a movement of the to-be-measured person is to be output, when said walking determining part determines that the to-be-measured person is currently walking and also said moving state determining part determines that a moving rate of the to-be-measured person in the horizontal direction is smaller than a predetermined value.

7. The measuring apparatus as claimed in claim 5, wherein:
said moving state determining part uses an output signal of a GPS unit when measuring the moving rate of the to-be-measured person.

8. The measuring apparatus as claimed in claim 1, wherein:
said measurement result output determining part determines that the measurement information concerning a movement of the to-be-measured person is to be output, when a moving rate of the to-be-measured person in a vertical direction is larger than a predetermined value, and also, a moving rate of the to-be-measured person in a horizontal direction is smaller than a predetermined value, during a time during which said walking determining part determines that the to-be-measured person is currently walking.

9. The measuring apparatus as claimed in claim 3, wherein:
said moving state determining part uses an output signal of an atmospheric pressure sensor when measuring the moving rate of the to-be-measured person.

10. A measuring method comprising:
a walking determining step of a walking determining part determining whether a to-be-measured person is currently walking, based on an output signal of a walking detecting part; and
a measurement result output determining step of a measurement result output determining part determining whether measurement information concerning a movement of the to-be-measured person obtained during a time during which said walking determining part determines that the to-be-measured person is currently walking is to be output, based on a moving rate of the to-be-measured person in a specific direction during the time.

11. The measuring method as claimed in claim 10, wherein:
in said measurement result output determining step, said measurement result output determining part determines that the measurement information concerning a movement of the to-be-measured person is to be output when a moving rate of the to-be-measured person in a vertical direction during a time during which said walking determining part determines that the to-be-measured person is currently walking in said walking determining step is larger than a predetermined value.

12. The measuring method as claimed in claim 10, wherein:
in said measurement result output determining step, said measurement result output determining part determines that the measurement information concerning a movement of the to-be-measured person is to be output when a moving rate of the to-be-measured person in a horizontal direction during a time during which said walking determining part determines that the to-be-measured person is currently walking in said walking determining step is smaller than a predetermined value.

13. The measuring method as claimed in claim 10, wherein:
in said measurement result output determining step, said measurement result output determining part determines that the measurement information concerning a movement of the to-be-measured person is to be output when a moving rate of the to-be-measured person in a vertical direction during a time during which said walking determining part determines that the to-be-measured person is currently walking in said walking determining step is larger than a predetermined value, and also, a moving rate of the to-be-measured person in a horizontal direction during the time is smaller than a predetermined value.

14. A computer readable information recording medium storing a program which, when executed by one or more processors of a measuring apparatus, carries out:
a walking determining step of determining whether a to-be-measured person is currently walking, based on an output signal of a walking detecting part; and
a measurement result output determining step of determining whether measurement information concerning a movement of the to-be-measured person obtained during a time during which it is determined in said walking determining step that the to-be-measured person is currently walking is to be output, based on a moving rate of the to-be-measured person in a specific direction during the time.

15. The computer readable information recording medium as claimed in claim 14, wherein:
in said measurement result output determining step, it is determined that the measurement information concerning a movement of the to-be-measured person is to be output when a moving rate of the to-be-measured person in a vertical direction during a time during which it is determined in said walking determining step that the to-be-measured person is currently walking is larger than a predetermined value.

16. The computer readable information recording medium as claimed in claim 14, wherein:
in said measurement result output determining step, it is determined that the measurement information concerning a movement of the to-be-measured person is to be output when a moving rate of the to-be-measured person in a horizontal direction during a time during which it is determined in said walking determining step that the to-be-measured person is currently walking is smaller than a predetermined value.

17. A measuring method for obtaining measurement information concerning a movement of a to-be-measured person, comprising the steps of:
a) obtaining the measurement information;
b) detecting an increase in the number of steps of the to-be-measured person to determine whether the to-be-measured person is currently walking;
c) detecting a moving amount of the to-be-measured person in a vertical direction; and
d) determining whether the measurement information is to be output based on the moving amount of the to-be-measured person in the vertical direction obtained when the to-be-measured person is currently walking.

18. A measuring method for obtaining measurement information concerning a movement of a to-be-measured person, comprising the steps of:
a) obtaining the measurement information;
b) detecting an increase in the number of steps of the to-be-measured person to determine whether the to-be-measured person is currently walking;
c) detecting a moving amount of the to-be-measured person in a horizontal direction; and
d) determining whether the measurement information is to be output based on the moving amount of the to-be-measured person in the horizontal direction obtained when the to-be-measured person is currently walking.

19. A portable terminal apparatus comprising:
a measuring part configured to obtain measurement information concerning a movement of a person who carries said portable terminal apparatus;
a walking determining part configured to determine a walking state of said person;
a moving state determining part configured to determine a moving state in a specific direction of said portable terminal apparatus; and
a measurement result output determining part configured to determine whether or not the measurement information obtained by said measuring part is to be output, based on a determination result of the walking state of said person obtained by said walking determining part and a determination result of the moving state of said portable terminal apparatus obtained by the moving state determining part.

20. The portable terminal apparatus as claimed in claim 19, further comprising:
a display part configured to display various sorts of information, wherein:
the measurement information, determined by said measurement result output determining part to output, is displayed by said display part.
